# EUROPEAN PATENT APPLICATION

(11) **EP 4 183 377 A1**
(43) Date of publication of application: **24.05.2023**
(21) Application number: 21209112.8
(22) Date of filing: 18.11.2021
(51) Int. Cl.: A61F 5/445, A61F 5/44

(54) **MEDICAL DEVICE FOR ABDOMINAL STOMAS**

(71) Applicant: Tecnoline S.p.A., 41033 Concordia Sulla Secchia, MO (IT)
(72) Inventor: PROVASI, Stefano, 41033 CONCORDIA SULLA SECCHIA MO (IT); BUGIANTELLA, Walter, 06073 CORCIANO PG (IT)
(74) Representative: Modiano, Micaela Nadia

(57) **Abstract**

A medical device for abdominal stomas (A) comprising a first orifice (B) and a second orifice (C), provided beforehand for communication to the outside respectively of an upstream part (E) and of a downstream part (F) of the intestine. The device (1) comprises a tube (2) which has at least one internal duct (3) and is bent into a U-shape in at least one active configuration, in order to define a first portion (2a) and a second portion (2b) which are connected by a bent portion (2c); the first portion (2a) and the second portion (2b) are configured, in use, for removable insertion in the upstream part (E) and in the downstream part (F) of the intestine and are associated with respective means for the flow control and conveyance of the substances in transit in the parts (E, F), configured to convey in use the substances within the duct (3) and to hinder their exit through the orifices (B, C).

## Description

The present invention relates to a medical device for abdominal stomas, particularly for ileostomies.

As is known, lateral ileostomy (or "lateral ileal fecal diversion", or "loop ileostomy") is a surgical procedure that is performed to divert intestinal transit externally, generally temporarily, protecting intestinal anastomoses performed downstream from the risk of contact with fecal matter and therefore from the possible forming of abscesses due to a failed consolidation of the anastomoses.

A temporary ileostomy can therefore be provided, for example, to rest intestinal tissues affected by a suture performed during a surgical procedure aimed at removing a tumor mass or to remedy severe forms of infection, both electively and urgently.

In practice, lateral ileostomy is provided surgically by bringing out of the abdominal cavity a loop of the small intestine (ileum), suturing it to the muscle fascia and to the skin and then cutting its wall at the level of the convexity so as to create an outward opening (indeed, a stoma), through which the fecal content is made to exit and is then collected in a bag: thus, as desired, the downstream portion of the intestine is preserved from fecal transit.

However, this procedure is not free from drawbacks.

Closing this type of ileostomy and restore normal intestinal transit requires a surgical procedure (recanalization) which obviously must be performed only when the part that is downstream of the intestine portion where the anastomosis/suture has been performed is consolidated (healed) for all purposes.

If in fact recanalization is performed with incorrect timing, for example in the presence of a failed consolidation of the downstream intestinal (ileoileal, ileocolic, ileorectal, colocolic, colorectal or coloanal) anastomosis, it leads to the foring of perianastomotic abscesses which require further surgical treatment with a new fecal diversion (ileostomy or colostomy) which can even become permanent.

It appears evident that such case is absolutely to be avoided, also because the risks entailed by any surgical procedure are associated with each operation (even more so if it is to be performed, as in these cases, under general anesthesia). Besides, the scientific literature in the field has by now documented extensively cases of significant complications which have affected patients subjected to these procedures.

On the other hand, it has now been established that it is often difficult to ascertain the complete healing and correct consolidation of anastomoses before proceeding with recanalization, thus incurring often the problems described above.

Finally, it should be noted that the presence of the ileostomy is often a cause of dehydration, inadequate absorption of nutrients, and other problems (for example dysionias), up to short bowel syndrome and high-output syndrome, which can lead to a severe deterioration of general condition (and sometimes even to death), such as to make intestinal recanalization surgery impossible or entailing very high risk.

The aim of the present invention is to solve the problems described above, providing a medical device that allows to verify the complete healing of the part of the intestine located downstream of a stoma.

Within this aim, an object of the invention is to provide a medical device that allows to verify the appropriateness of proceeding with a surgical recanalization procedure, having been able to ascertain beforehand the correct consolidation of the anastomoses in a practical and easy manner.

Another object of the invention is to provide a medical device which, by temporarily restoring physiological intestinal transit, ensures adequate physiological absorption of nutrients and liquids.

Another object of the invention is to provide a medical device that ensures high reliability in operation.

Another object of the invention is to propose a medical device that uses a technical and structural architecture that is alternative to those of medical devices of the known type.

Not least object of the invention is to provide a medical device that can be obtained easily starting from commonly commercially available elements and materials.

Another object of the invention is to provide a medical device that has low costs and is safe in application.

This aim and these and other objects which will become better apparent hereinafter are achieved by a medical device according to claim 1 and by a kit according to claim 7.

Further characteristics and advantages of the invention will become better apparent from the description of a preferred but not exclusive embodiment of the medical device according to the invention, illustrated by way of non-limiting example in the accompanying drawings, wherein:

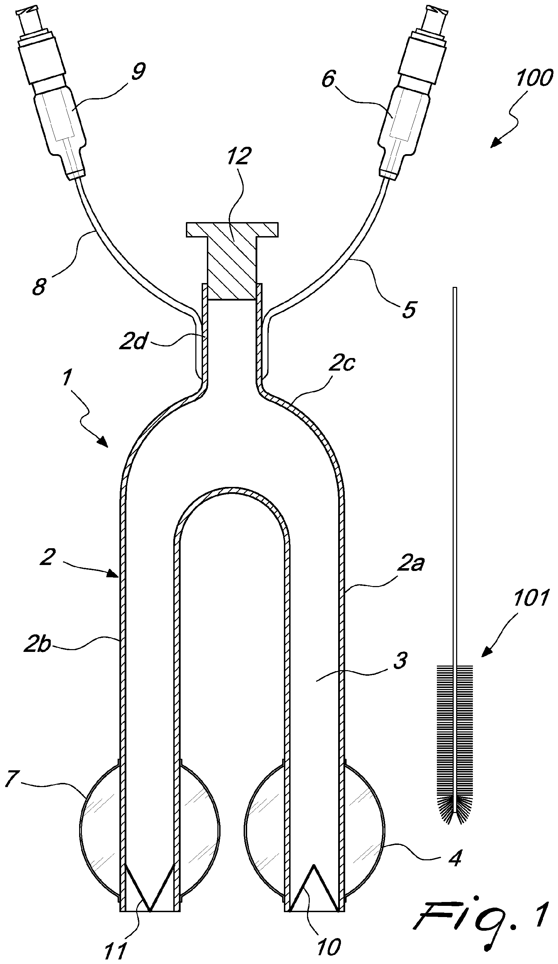

Figure 1 is a front elevation view of the device and of the kit according to the invention, with the device in cross-section along a longitudinal plane;

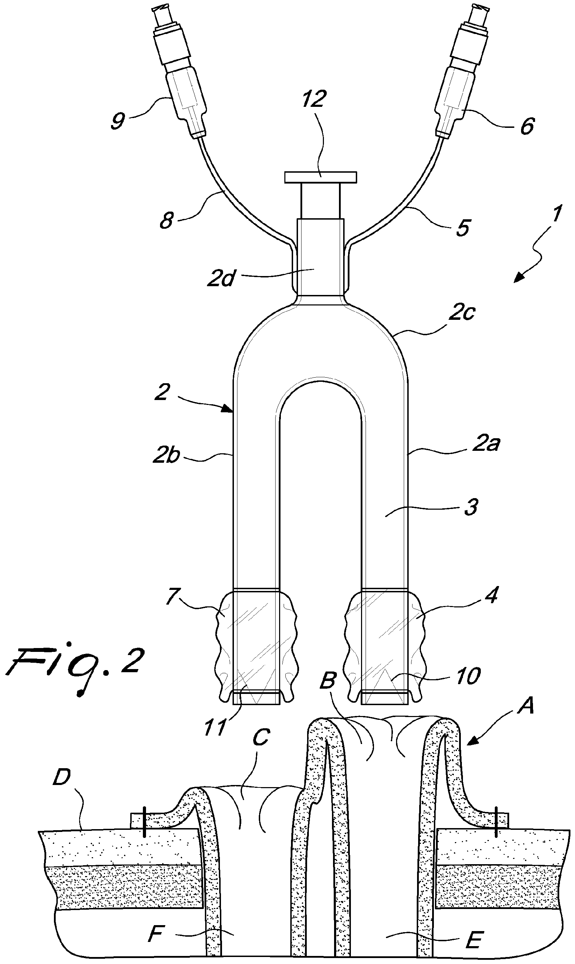

Figures 2 and 3 are sequential front elevation views of the use of the device according to the invention,
Figure 4 is a perspective view of a stoma on which it is possible to use the device and the kit according to the invention.

With particular reference to the figures, the reference numeral 1 generally designates a medical device for abdominal stomas A (although the use of said device 1 for other and different stomas A is not excluded).

In per se known manners, the abdominal stomas A for which the device 1 is intended to find an application comprise (or more simply consist of) a first orifice B and a second orifice C provided beforehand, typically along the abdominal wall D (or also another body region) of a patient, in order to connect to the outside respectively an upstream part E (afferent loop) and a downstream part F (efferent loop) of the intestine (of the same patient, of course). The orifices B, C can be arranged superficially, and therefore be visible from the outside as in the accompanying figures, but the possibility is not excluded that only a hole is visible from the outside which leads to the two orifices B, C located deeper, at for example a branching from which the upstream part E and the downstream part F in any case extend.

In this regard it is useful to specify that in the present description the terms "upstream", "downstream", "upstream of" and "downstream of" are terms used according to common usage and therefore refer to what precedes ("upstream") or follows "("downstream") a reference (the orifices B, C) with respect to the direction of motion of the substances inside the intestine (physiologically from the mouth toward the anus).

Therefore, the (fecal) substances in transit along the intestine (which arrive from the upstream part E) can exit from the first orifice B and this allows to rest the downstream part F, typically to give the previously provided anastomoses the time to consolidate.

The stoma A and the two orifices B, C are typically (but not necessarily) provided by bringing out of the abdominal cavity an intestinal loop, suturing it to the skin and then cutting into its lateral surface in order to indeed obtain the orifices B, C.

In any case, the details regarding the way in which the stoma A is provided, as well as the purposes for which it is obtained, are well-known in the art and therefore will not be dwelt upon further in the present description.

It is specified moreover that in the preferred application the use of the device 1 is provided in relation to a stoma A obtained following lateral ileostomy (or lateral ileal fecal diversion, or loop ileostomy), performed for any purpose, but its use also for other abdominal stomas A (or of another type) is not excluded.

According to the invention, the device 1 comprises a tube 2 which has at least one internal duct 3 and is bent into a U-shape in at least one active configuration (as in the accompanying figures).

In this manner (at least in the active configuration) the tube 2 forms a first portion 2a and a second portion 2b which are connected by a bent portion 2c (the portions 2a, 2b, 2c forming typically the entire longitudinal extension of the tube 2).

The first portion 2a and the second portion 2b are configured in use for removable insertion in the upstream part E and in the downstream part F of the intestine; therefore, this means that at least the shape, the materials and the dimensions of the first portion 2a and of the second portion 2b are chosen so as to indeed allow insertion in the parts E, F of the intestine. Moreover, in order to ensure higher versatility of use to the invention, tubes 2, and therefore first portions 2a and second portions 2b, having different diameters are provided in anticipation of use on adult patients or children.

It is specified that the tube 2 can be rigid and therefore be constantly bent into a U-shape or can be at least partially flexible, so as to allow the surgeon to bend it into a U-shape at least in use or in any case in order to be able to adapt it to the shape of the parts E, F of the intestine. In the case of an at least partially flexible tube 2, the surgeon can in fact divaricate to a greater or smaller extent the first portion 2a and the second portion 2b, which can also bend inside the parts E, F in order to follow their shape.

The curvature and in general the shape of the bents portion 2c may be any and preferably they are chosen such (or can be shaped so) as to determine the smallest possible space occupation.

It is specified that where, in the present description, the expression "in use" is used, it should be understood as the condition that indeed occurs when the first portion 2a and the second portion 2b of the tube 2 (which is bent into a U-shape) are inserted in the respective parts E, F of the intestine (the bent portion 2c remaining instead outside).

Moreover, according to the invention the first portion 2a and the second portion 2b are associated with respective means for flow control and conveyance of the substances (in particular fecal matter) in transit in the parts E, F. Said means (of which a practical embodiment will be provided in the pages that follow, and which are therefore certainly part of the device 1 according to the invention) are configured to convey in use the substances in transit within the duct 3 and to hinder their exit through the orifices B, C.

In practice, therefore, after inserting the first portion 2a in the upstream part E and the second portion 2be in the downstream part F, one obtains a temporary recanalization and a restoration of the normal flow of the substances in transit in the intestine, which from the upstream part E are conveyed into the duct 3, from which they exit toward the downstream part F, being then able to continue their path toward the anus.

This allows therefore to verify (in a practical and easy manner) whether the body of the patient and in particular the intestine has regained its normal functionality, so as to proceed with the surgical procedure for intestinal recanalization, which is normally provided to restore the correct functionality of said intestine, only if the verification has a positive outcome. Otherwise, one may simply remove the device 1 and keep the stoma A active.

The intended aim is therefore achieved already at this point.

In particular, in the preferred embodiment, shown in the accompanying figures by way of non-limiting example of the application of the invention, the flow control and conveyance means comprise a first annular enclosure 4 which is coupled externally to the lateral surface of the first portion 2a and can be inflated selectively, for hermetic adhesion, in use, to the internal wall of the upstream part E of the intestine.

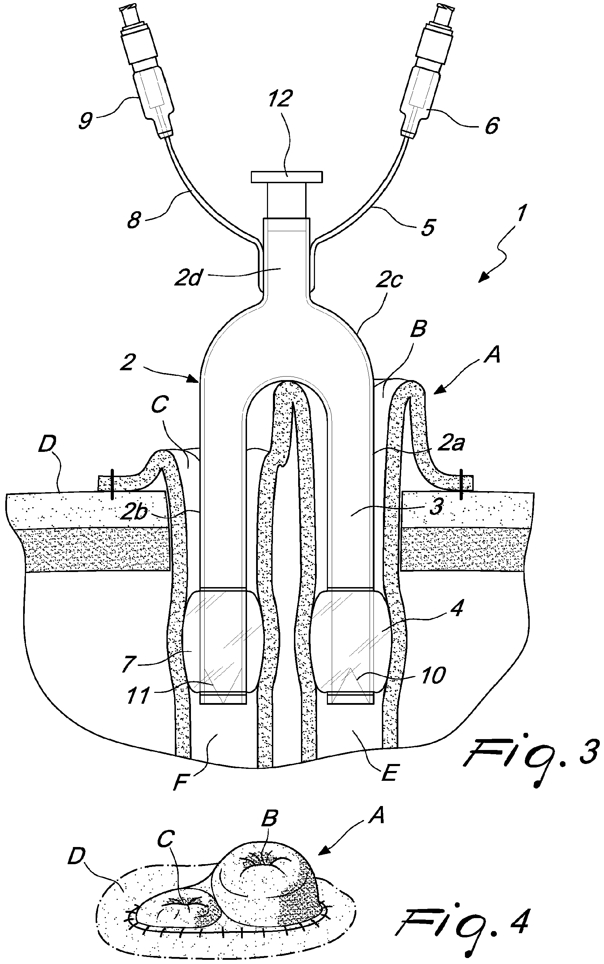

As shown by the accompanying figures, the first enclosure 4 is in practice a sort of balloon which is wrapped around a (preferably terminal) segment of the first portion 2a: once inflated, it expands until it presses against the internal wall of the upstream part F (Figure 3). In addition to performing a useful function of anchoring to the intestine, which ensures greater stability to the device 1, by adhesion to the internal wall of the intestine the first enclosure 4 indeed prevents the substances in transit from seeping along the interspace between the tube 2 and the intestine and then exiting in an unwanted manner through the first orifice B.

In practice, the flow control and conveyance means comprise a first inflation line 5 which is or can be associated with a source of fluid in overpressure (for example a manually operated pump), to indeed inject air into the first enclosure 4 (which communicates with the first line 5).

At least one fraction of the first line 5 can (preferably) be integrated in the thickness of the tube 2 or can be arranged inside it or outside it (being extended in any case substantially parallel to the axis of the duct 3), to then extend beyond the bent portion 2c (which in use is typically kept outside the abdominal wall D of the patient) until it ends with a first connector 6 for connection to the source of fluid in overpressure.

With further reference to the preferred embodiment shown in the accompanying figures, the flow control and conveyance means comprise a second annular enclosure 7 which is coupled externally to the lateral surface of the second portion 2b and can be inflated selectively, for hermetic adhesion, in use, to the internal wall of the downstream part F of the intestine.

In practice, the second annular enclosure 7 can be of the type of the first enclosure 4 and can perform a similar role: the second enclosure 7 also is a sort of balloon which is wrapped around a (preferably terminal) segment of the second portion 2b, which, once inflated (Figure 3), expands and presses against the internal wall of the downstream part F. Again, therefore, the second enclosure 7 also performs first of all a useful function of anchoring to the intestine and most of all prevents the substances contained in the downstream part F from being able to rise along the interspace between the tube 2 and the intestine until they exit in an unwanted manner through the second orifice C.

As a further analogy to what has been observed for the first enclosure 4, the device 1 is provided with a second inflation line 8 (with a second connector 9) which is or can be associated with a source of fluid in overpressure (the same one as the first line 5 or a different one), in order to inject air into the second enclosure 7 (communicating with the second line 8). The additional constructive details can of course be the same ones described above for the first line 5 or different ones.

Usefully, the device 1 can comprise a first one-way valve 10, which is located at the free end of the first portion 2a (oppositely with respect to the bent portion 2c) and is configured in use for unidirectional clearance to transit of the substances from the upstream part E of the intestine to the first portion 2a. In other words, therefore, the first one-way valve 10 does not allow transit in the opposite direction (from the first portion 2a to the upstream part E).

The first one-way valve 10 is a further assurance that the movement of substances occurs exclusively in the desired direction.

Favorably, the device 1 can comprise a second one-way valve 11, which is located at the free end of the second portion 2b (oppositely with respect to the bent portion 2c) and is configured in use for unidirectional clearance for the transit of substances from the second portion 2b to the downstream part F of the intestine. In other words, therefore, the second one-way valve 11 does not allow transit in the opposite direction (from the downstream part F to the second portion 2b).

The second one-way valve 11, therefore, also is a further assurance that the movement of substances occurs exclusively in the desired direction.

In practice, the one-way valves 10, 11 can be of any type (including known ones), chosen among those that the person skilled in the art would know how to deem adequate for the purposes outlined above.

Advantageously, the tube 2 can comprise an outward opening at the convexity of the bent portion 2c, i.e., at the part of the external surface of the bent portion 2c which in use is directed oppositely with respect to the intestine of the patient (and, at least in the active configuration, to the first portion 2a and to the second portion 2b). In particular, as in the accompanying figures, the opening can be provided on a tubular tab 2d which extends transversely (with respect to the longitudinal extension of the tube 2) from the convexity of the bent portion 2c.

The opening is normally closed by a plug 12 which can be removed selectively for access from the outside to the duct 3. This is useful for example in order to be able to perform operations for cleaning, emptying or obstruction removal, even when the tube 2 is inserted with the first portion 2a and the second portion 2b in the respective parts E, F of the intestine.

It is specified that preferably the tubular tab 2d and/or the plug 12 are chosen with minimal dimensions, chosen so as to allow these components to perform the functions described above but also such as to contain as much as possible the overall space occupation of the device 1.

The subject matter of the present description and of the protection that is claimed thereby is therefore, first of all, a medical device 1 that has the particularities described so far.

Likewise, the present description claims protection also on a kit 100 for abdominal stomas A, wherein said stomas A are of the type already described and therefore comprise a first orifice B and a second orifice C previous beforehand in order to establish communication with the outside respectively of an upstream part E and of a downstream part F of the intestine.

According to the invention, the kit 100 comprises at least one device 1 according to what has already been described and a (preferably flexible) cleaning brush 101, which can be inserted in at least one between the first portion 2a and the second portion 2b in order to remove any obstructions. Preferably, in any case, the cleaning brush 101 can be chosen suitable for its insertion both in the first portion 2a and in the second portion 2b.

The cleaning brush 101 is sized so that in use it cannot exit from the corresponding free end of the tube 2 (of the first portion 2a and/or of the second portion 2b) and therefore cannot damage the neighboring anatomical tissues (of the intestinal wall).

In particular, the cleaning brush 101 can be shorter than or as long as the distance (measured longitudinally) between the opening and at least one between the first portion 2a and the second portion 2b.

The use of the device (and of the kit) according to the invention is as follows.

In the presence of a stoma A aimed at ensuring protection of an anastomosis (for example a colorectal or coloanal one), the device 1 can be used to verify the consolidation and integrity of said anastomosis before performing a surgical procedure for permanent recanalization.

In order to perform this verification by means of the device 1, the surgeon (or other qualified individual) must insert the first portion 2a of the tube 2 in the first orifice B and therefore in the upstream part E of the intestine (in the direction of arrival of the intestinal content) and the second portion 2b of the tube 2 in the second orifice C and therefore in the downstream part F of the intestine (in the direction of destination of the intestinal content). The tube 2, which is bent into a U-shape at least in the active configuration described herein, remains in practice outside with the bent portion 2c (and with the opening closed by the plug 12) which mutually connects the first portion 2a and the second portion 2b.

The enclosures 4, 7 provided on each free end allow (when inflated, as in the Figure 3) to convey in the appropriate manner the intestinal transit toward the duct 3 and therefore toward the anus, thus ensuring physiological transit from the upstream part E to the downstream part F, avoiding the outflow of the intestinal content outside the stoma A through the orifices B, C (through the first orifice B in particular).

Thus, with a small number of simple operations physiological intestinal transit through the small intestine and then through the large intestine is in practice restored (at least temporarily) (the fecal substances can again travel along the downstream part F and reach the anus): this allows to verify in a practical and easy manner the complete healing of the entire segment of the intestine located downstream of the stoma A and to ascertain the correct consolidation of the anastomoses, so as to evaluate beforehand and effectively the opportuneness of proceeding with the surgical recanalization procedure (by means of which it is possible to permanently restore the regular operation of the intestine). If the verification yields a negative outcome, it is sufficient to remove the device 1 and continue with the use of the stoma A for external evacuation of the fecal substances.

The introduction of the device 1 and therefore the restoration of normal intestinal transit allow physiological absorption of nutrients and liquids, avoiding the problems of dehydration, dysionia and nutritional deficits that sometimes are observed in patients in which a stoma A has been provided.

When one wishes to remove the device 1 (to reactivate the normal operation of the stoma A and optionally start the surgical recanalization procedure) it is sufficient to deflate the enclosures 4, 7 and extract the first portion 2a and the second portion 2b from the parts E, F. Again, there are few and simple operations.

The valves 10, 11 also contribute to ensure the correct movement of the contents of the intestine.

The device 1 is therefore certainly effective and reliable, also by virtue of the simplicity that characterizes it (which is also an assurance of low cost).

The device 1 is indicated for the temporary recanalization of lateral ileostomy (or also of others stomas A) both in adults and in children (it is sufficient to vary the size of the tube 2 and in particular its diameter in order to be able to use it in ileal loops of any size).

The cleaning brush 101 which can be supplied with the device 1 (in a kit 100) allows to remove obstructions in a practical and easy manner even during the use of said device 1.

In practice it has been found that the medical device 1 according to the invention fully achieves the intended aim and objects, since resorting to a tube 2 that is bent into a U-shape and can be inserted with a first portion 2a and a second portion 2b respectively in the upstream part E and in the downstream part F of the intestine allows, by virtue also of the flow control and conveyance means for the substances in transit, to temporarily restore intestinal transit and therefore verify the complete healing of the part of the intestine located downstream of a stoma A.

The invention thus conceived is susceptible of numerous modifications and variations, all of which are within the scope of the accompanying claims; all the details may furthermore be replaced with other technically equivalent elements.

In the embodiments shown, individual characteristics, given in relation to specific examples, may actually be interchanged with other different characteristics that exist in other embodiments.

In practice, the materials used, as well as the dimensions, may be any according to the requirements and the state of the art.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A medical device for abdominal stomas (A), stomas (A) comprising a first orifice (B) and a second orifice (C), provided beforehand for communication to the outside respectively of an upstream part (E) and of a downstream part (F) of the intestine, **characterized in that** it comprises a tube (2) which has at least one internal duct (3) which is bent into a U-shape in at least one active configuration, in order to define a first portion (2a) and a second portion (2b) which are connected by a bent portion (2c), said first portion (2a) and said second portion (2b) being configured, in use, for removable insertion in the upstream part (E) and in the downstream part (F) of the intestine and being associated with respective means for the flow control and conveyance of the substances in transit in the parts (E, F), configured to convey in use the substances within said duct (3) and to hinder their exit through the orifices (B, C).

2. The medical device according to claim 1, **characterized in that** said flow control and conveyance means comprises a first annular enclosure (4) which is coupled externally to the lateral surface of said first portion (2a) and can be inflated selectively, for hermetic adhesion, in use, to the internal wall of the upstream part (E) of the intestine.

3. The medical device according to claim 1 or 2, **characterized in that** said flow control and conveyance means comprise a second annular enclosure (7) which is coupled externally to the lateral surface of said second portion (2b) and can be inflated selectively, for hermetic adhesion, in use, to the internal wall of the downstream part (F) of the intestine.

4. The medical device according to one or more of the preceding claims, **characterized in that** it comprises a first one-way valve (10), arranged at the free end of said first portion (2a) and configured in use for unidirectional clearance for the transit of the substances from the upstream part (E) of the intestine to said first portion (2a).

5. The medical device according to one or more of the preceding claims, **characterized in that** it comprises a second one-way valve (11), located at the free end of said second portion (2b) and configured in use for unidirectional clearance for the transit of the substances from said second portion (2b) to the downstream part (F) of the intestine.

6. The medical device according to one or more of the preceding claims, **characterized in that** said tube (2) comprises an outward opening at the convexity of said bent portion (2c), which is directed in use oppositely with respect to the intestine of the patient, said opening being normally closed by a plug (12) which can be removed selectively for access to said duct (3) from outside.

7. A kit for abdominal stomas, stomas (A) comprising a first orifice (B) and a second orifice (C) provided beforehand for communication with the outside respectively of an upstream part (E) and of a downstream part (F) of the intestine, **characterized in that** it comprises at least one device (1) according to one or more of the preceding claims and a cleaning brush (101), which can be inserted in at least one between said first portion (2a) and said second portion (2b) for the removal of any obstructions.

8. The kit according to claim 7, **characterized in that** said cleaning brush (101) is shorter than or as long as the distance between said opening and at least one between said first portion (2a) and said second portion (2b).
